# EUROPEAN PATENT SPECIFICATION

(11) **EP 4 324 402 B1**
(45) Date of publication and mention of the grant of the patent: **11.11.2026**
(21) Application number: 22788257.8
(22) Date of filing: 02.03.2022
(51) Int. Cl.: G01S 7/52, G01S 15/89, A61B 8/08, A61B 8/00, A61B 5/107

(54) **ULTRASONIC VOLUME MEASUREMENT DEVICE AND ULTRASONIC VOLUME MEASUREMENT SYSTEM COMPRISING SAME**
ULTRASCHALLVOLUMENMESSVORRICHTUNG UND ULTRASCHALLVOLUMENMESSSYSTEM DAMIT
DISPOSITIF DE MESURE DE VOLUME ULTRASONORE ET SYSTÈME DE MESURE DE VOLUME ULTRASONORE LE COMPRENANT

(30) Priority: 16.04.2021 KR 20210049928
(43) Date of publication of application: 21.02.2024
(73) Proprietor: Edgecare Inc., Seoul 03911 (KR); Sogang University Research & Business Development Foundation, Seoul 04107 (KR)
(72) Inventor: YOO, Yang Mo, Goyang-si Gyeonggi-do 10374 (KR)
(74) Representative: Frenkel, Matthias Alexander
(86) International application number: PCT/KR2022/002920
(87) International publication number: WO 2022/220400

(56) References cited:
- JP-A- 2001 198 122
- KR-A- 20130 139 704
- KR-A- 20150 027 482
- KR-A- 20200 128 871
- KR-A- 20210 032 866
- US-A- 5 450 850

## Description

### [Technical Field]

The present disclosure relates to an ultrasonic volume measurement device and an ultrasonic volume measurement system including the same.

### [Background Art]

The use of one-dimensional array to measure the volume of a certain area inside the human body may have many difficulties in measuring the exact volume. Recently, various studies have been conducted to solve this problem. US 5 450 850 A discloses a cardiac function examination system comprising a device for measuring the amount of blood ejected from the ventricle to the aorta based on reflected ultrasonic pulses, a device for measuring the aorta or thick artery diameter based on reflected ultrasonic pulses, a device for inputting at least one of maximum and minimum blood pressure values, a device for computing a maximum ventricular volume or an ejection fraction, and a device for acquiring parameters such as cardiac volume or aorta pressure parameter regarding cardiac function based on the measured values of the ejection amount, the blood vessel diameter, and the ejection fraction, or any one of these, and the input pressure values.

### [Disclosure]

### [Technical Problem]

An object of the present disclosure is to provide an ultrasonic volume measurement device which includes multiple second directional elements arranged in a second direction perpendicular to a first direction in which a first ultrasonic array is disposed and which is capable of accurately determining volume information of a measurement site included in an image region using a second ultrasonic array disposed on one side of the first ultrasonic array.

### [Technical Solution]

The invention is defined by the appended claims.

### [Advantageous Effects]

According to the present disclosure as described above, the following effects are achieved.

The ultrasonic volume measurement device according to the present disclosure includes multiple second directional elements arranged in the second direction perpendicular to the first direction in which the first ultrasonic array is disposed and may accurately determine volume information of a measurement site included in an image region using the second ultrasonic array disposed on one side of the first ultrasonic array.

.In addition, other features and advantages of the present disclosure may be newly understood through embodiments of the present disclosure.

### [BRIEF DESCRIPTION OF THE DRAWINGS]

FIG. 1 is a diagram illustrating an ultrasonic volume measurement device according to embodiments of the present disclosure.
FIG. 2 is a diagram illustrating an embodiment of the ultrasonic volume measurement device of FIG. 1.
FIG. 3 is a diagram illustrating an operation of the ultrasonic volume measurement device of FIG. 1.
FIG. 4 is a diagram illustrating another operation of the ultrasonic volume measurement device of FIG. 1.
FIG. 5 is a diagram illustrating an image providing unit included in the ultrasonic volume measurement device of FIG. 1.
FIGS. 6 and 7 are diagrams illustrating first ultrasonic images generated using a first ultrasonic array included in the ultrasonic volume measurement device of FIG. 1.
FIGS. 8 and 9 are diagrams illustrating second ultrasonic images generated using a second ultrasonic array included in the ultrasonic volume measurement device of FIG. 1.
FIG. 10 is a diagram illustrating a steering angle applied to the ultrasonic volume measurement device of FIG. 1.
FIG. 11 is a diagram illustrating an operating mode of the ultrasonic volume measurement device of FIG. 1.
FIG. 12 is a diagram illustrating an example of a steering angle applied to the ultrasonic volume measurement device of FIG. 1.
FIG. 13 is a diagram illustrating an ultrasonic imaging device according to embodiments of the present disclosure.
FIG. 14 is a diagram illustrating an operation of an array controller included in the ultrasonic imaging device of FIG. 13.
FIG. 15 is a flowchart illustrating an operating method of an ultrasonic device according to embodiments of the present disclosure.
FIG. 16 is a flowchart illustrating an operating method of an ultrasonic imaging device according to embodiments of the present disclosure.

### [Best Mode]

In adding reference numerals for elements in each figure, it should be noted that like reference numerals already used to denote like elements in other figures are used for elements wherever possible.

Terms described in the present disclosure may be understood as follows.

Singular forms "a", "an" and "the" in the present disclosure are intended to include the plural forms as well, unless the context clearly indicates otherwise, and such components must not be understood as being limited to the above terms.

It should be further understood that the terms "comprises", "comprising,", "has", "having", "includes" and/or "including", when used herein, specify the presence of stated features, integers, steps, operations, elements, and/or components, but do not preclude the presence or addition of one or more other features, integers, steps, operations, elements, components, and/or groups thereof.

Hereinafter, embodiments of the present disclosure designed to solve the above problems will be described in detail with reference to the accompanying drawings.

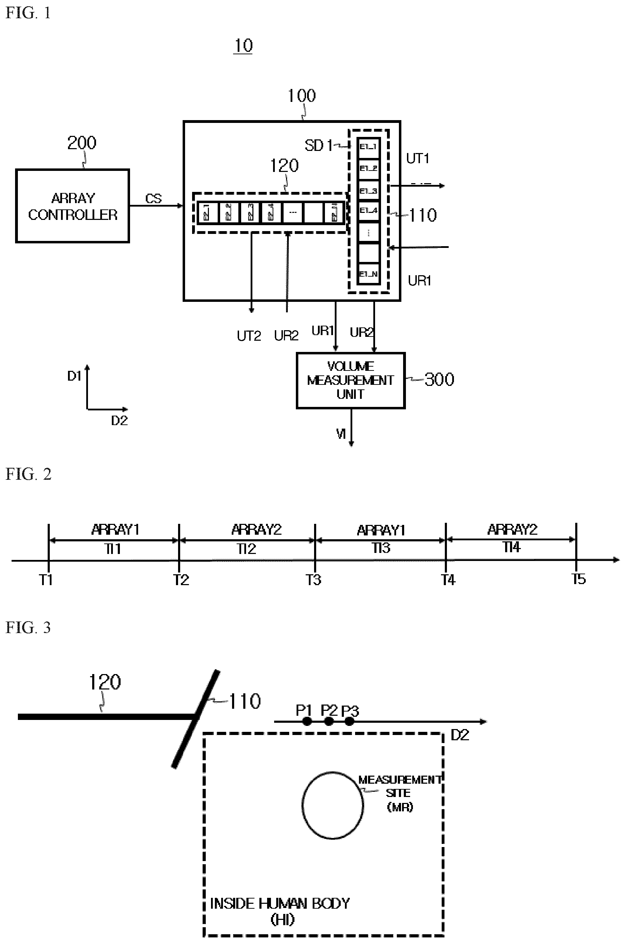

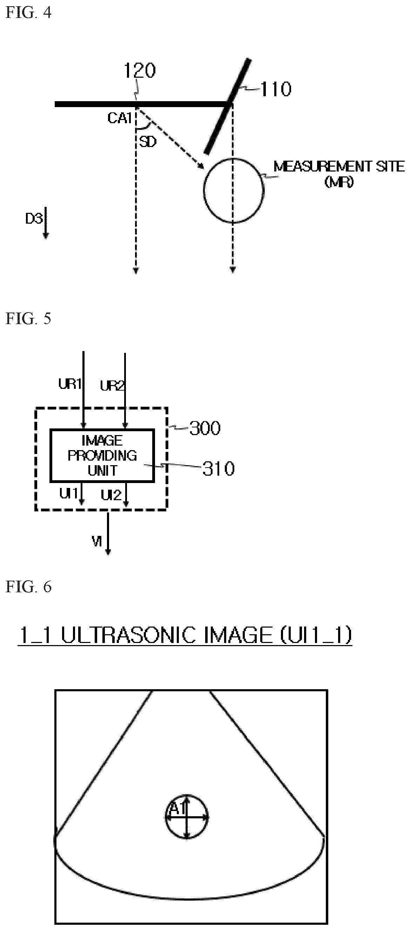

FIG. 1 is a diagram illustrating an ultrasonic volume measurement device according to embodiments of the present disclosure, FIG. 2 is a diagram illustrating an embodiment of the ultrasonic volume measurement device of FIG. 1, FIG. 3 is a diagram illustrating an operation of the ultrasonic volume measurement device of FIG. 1, and FIG. 4 is a diagram illustrating another operation of the ultrasonic volume measurement device of FIG. 1.

Referring to FIGS. 1 to 4, an ultrasonic volume measurement device 10 according to an embodiment of the present disclosure includes a first ultrasonic array 110, a second ultrasonic array 120, an array controller 200, and a volume measurement unit 300.

The first ultrasonic array 110 includes multiple first directional elements arranged in a first direction D1 and may transmit a first ultrasonic transmission signal UT1. For example, multiple first directional elements may include a 1_1 element E1_1 and 1_2 element E1_2 to 1_N element E1_N. The 1_1 element E1_1 and the 1_2 element E1_2 to the 1_N element E1_N may be arranged in the first direction D1, and the first ultrasonic array 110 may transmit a first ultrasonic transmission signal UT1 using the 1_1 element E1_1 and the 1_2 element E1_2 to the 1_N elements E1_N. The second ultrasonic array 120 includes multiple second directional elements arranged in a second direction D2 perpendicular to the first direction D1, transmit a second ultrasonic transmission signal UT2, and may be disposed on one side SD1 of the first ultrasonic array 110. For example, multiple second directional elements may include a 2_1 element E2_1 and 2_2 element to 2_N element E2_N. The 2_1 element E2_1 and the 2_2 element to the 2_N element E2_N may be arranged in the second direction D2 perpendicular to the first direction D1, and the second ultrasonic array 120 may be vertically disposed on one side SD1 of the first ultrasonic array 110. The second ultrasonic array 120 may transmit a second ultrasonic transmission signal UT2 using the 2_1 element E2_1 and the 2_2 element to the 2_N elements E2_N.

The array controller 200 provides control signals for controlling the first ultrasonic array 110 and the second ultrasonic array 120. The volume measurement unit 300 provides volume information VI of a measurement site MR included in an image region generated based on a first ultrasonic reception signal UR1 received by the first ultrasonic array 110 and a second ultrasonic reception signal UR2 received by the second ultrasonic array 120.

In an embodiment, when the first ultrasonic array 110 and the second ultrasonic array 120 are disposed at a certain position of a human body, the first ultrasonic array 110 and the second ultrasonic array 120 are sequentially turned on to receive the first ultrasonic reception signal UR1 and the second ultrasonic reception signal UR2. For example, multiple times may include a first time T1, a second time T2, a third time T3, a fourth time T4, and a fifth time T5. A time interval from the first time T1 to the second time T2 may be a first time interval TI1, and a time interval from the second time T2 to the third time T3 may be a second time interval TI2. In addition, a time interval from the third time T3 to the fourth time T4 may be a third time interval TI3, and a time interval from the fourth time T4 to the fifth time T5 may be a fourth time interval TI4.

For example, the center of the first ultrasonic array 110 may be disposed at a first point P1 during the first time interval TI1 and the second time interval TI2. In this case, during the first time interval TI1, the ultrasonic volume measurement device 10 drives the first ultrasonic array 110 based on a first control signal CS1 to transmit the first ultrasonic transmission signal UT1 and receive the first ultrasonic reception signal UR1 reflected from an object. Meanwhile, during the first time interval TI1, the ultrasonic volume measurement device 10 turns off the second ultrasonic array 120 based on a second control signal CS2 and not drive the second ultrasonic array 120. During the second time interval TI2, the ultrasonic device turns off the first ultrasonic array 110 based on the first control signal CS1 and not drive the first ultrasonic array 110, while the ultrasonic device drives the second ultrasonic array 120 based on the second control signal CS2 to transmit the second ultrasonic transmission signal UT2 and receive the second ultrasonic reception signal UR2 reflected from the object. In an embodiment, the volume measurement device according to the present disclosure may be located at the first point P1 of the human body during the first time interval TI1 and the second time interval TI2 to acquire both the first ultrasonic image UI1 and the second ultrasonic image UI2 of the object imaged at the first point P1.

The first ultrasonic image UI1 and the second ultrasonic image UI2 may be stored in a database, and the volume measurement unit 300 may provide the volume information VI of the measurement site MR included in the image region generated based on the first ultrasonic reception signal UR1 received by the first ultrasonic array 110 and the second ultrasonic reception signal UR2 received by the second ultrasonic array 120.

Thereafter, the center of the first ultrasonic array 110 may be disposed at a second point P2 during the third time interval TI3 and the fourth time interval TI4. In this case, during the third time interval TI3, the ultrasonic volume measurement device 10 may drive the first ultrasonic array 110 based on the first control signal CS1 to transmit the first ultrasonic transmission signal UT1 and receive the first ultrasonic reception signal UR1 reflected from the object. Meanwhile, during the third time interval TI3, the ultrasonic volume measurement device 10 may turn off the second ultrasonic array 120 based on the second control signal CS2 and not drive the second ultrasonic array 120. During the fourth time interval TI4, the ultrasonic volume measurement device 10 may turn off the first ultrasonic array 110 based on the first control signal CS1 and not drive the first ultrasonic array 110, and meanwhile, the ultrasonic volume measurement device 10 may drive the second ultrasonic array 120 based on the second control signal CS2 to transmit the second ultrasonic transmission signal UT2 and receive the second ultrasonic reception signal UR2 reflected from the object. In an embodiment, the volume measurement device according to the present disclosure may be located at the second point P2 of the human body during the third time interval TI3 and the fourth time interval TI4 and acquire both the first ultrasonic image UI1 and the second ultrasonic image UI2 of the object captured at the second point P2. The first ultrasonic image UI1 and the second ultrasonic image UI2 may be stored in a database, and the volume measurement unit 300 may provide the volume information VI of the measurement site MR included in the image region generated based on the first ultrasonic reception signal UR1 received by the first ultrasonic array 110 and the second ultrasonic reception signal UR2 received by the second ultrasonic array 120.

In an embodiment, the second ultrasonic array 120 may transmit the second ultrasonic transmission signal UT2 having a steering angle SD corresponding to a predetermined angle to the measurement site MR included in the image region and receive the second ultrasonic reception signal UR2 reflected from the measurement site MR. For example, in order to acquire an image including the measurement site MR using the second ultrasonic array 120, a delay applied to multiple second directional elements included in the second ultrasonic array 120 may be changed. When the delay applied to the second directional elements changes, the steering angle SD of the second ultrasonic array 120 may be changed.

The ultrasonic volume measurement device 10 according to the present disclosure may include multiple second directional elements arranged in the second direction D2 perpendicular to the first direction D1 in which the first ultrasonic array 110 is disposed, and may accurately identify the volume information VI of the measurement site MR included in the image region using the second ultrasonic array 120 disposed on one side SD1 of the first ultrasonic array 110.

In the above, the operation of alternately scanning the first ultrasonic array 110 and the second ultrasonic array 120 by dividing time is described. However, the ultrasonic device 10 according to the present disclosure may transmit an ultrasonic transmission signal using the first ultrasonic array 110 and the second ultrasonic array 120 simultaneously, receive an ultrasonic reception signal reflected from the object using the first ultrasonic array 110 and the second ultrasonic array 120 simultaneously, and perform signal processing, thereby acquiring an ultrasonic image.

In addition, the ultrasonic volume measurement device 10 according to an embodiment of the present disclosure may measure a length of the deepest part of a bladder using the first ultrasonic array 110 and measure a height of an image acquired from the bladder using the second ultrasonic array 120 disposed at an angle of 90 degrees with the first ultrasonic array 110, thereby measuring a three-dimensional volume of the bladder.

FIG. 5 is a diagram illustrating an image providing unit included in the ultrasonic volume measurement device of FIG. 1, FIGS. 6 and 7 are diagrams illustrating first ultrasonic images generated using a first ultrasonic array included in the ultrasonic volume measurement device of FIG. 1, and FIGS. 8 and 9 are diagrams illustrating second ultrasonic images UI2 generated using a second ultrasonic array included in the ultrasonic volume measurement device of FIG. 1.

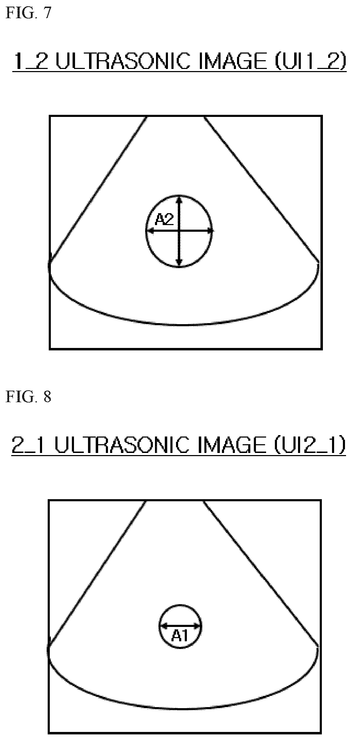

Referring to FIGS. 1 to 9 , the ultrasonic volume measurement device 10 may further include an image providing unit 310. The image providing unit 310 may provide the first ultrasonic image UI1 corresponding to the first ultrasonic reception signal UR1 and the second ultrasonic image UI2 corresponding to the second ultrasonic reception signal UR2 at each array position in which the first ultrasonic array 110 and the second ultrasonic array 120 are arranged. For example, when the center of the first ultrasonic array 110 is disposed at the first point P1, the 1_1 ultrasonic image U1_1 generated based on the first ultrasonic reception signal UR1 received by the first ultrasonic array 110 may be illustrated as shown in FIG. 6, and the height and width of the measurement site MR shown in FIG. 6 may be A1. Thereafter, the second ultrasonic transmission signal UT2 may be transmitted to the measurement site MR using the second ultrasonic array 120, and the 2_1 ultrasonic image U2_1 generated based on the second ultrasonic reception signal UR2 received from the measurement site MR may be illustrated as shown in FIG. 8, and the width of the measurement site MR shown in FIG. 8 may be A1.

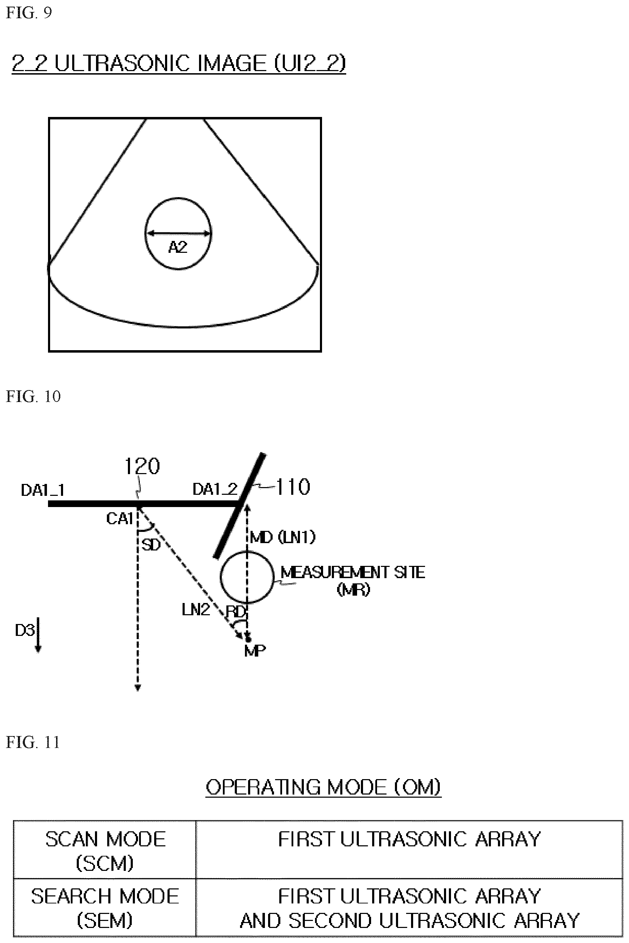

Thereafter, the first ultrasonic array 110 and the second ultrasonic array 120 may move to the second point P2. When the center of the first ultrasonic array 110 is located at the second point P2, the 1_2 ultrasonic image U1_2 generated based on the first ultrasonic reception signal UR1 received by the first ultrasonic array 110 may be illustrated as shown in FIG. 7, and the height and width of the measurement site MR shown in FIG. 7 may be A2. Thereafter, the second ultrasonic transmission signal UT2 may be transmitted to the measurement site MR using the second ultrasonic array 120, the 2_2 ultrasonic image U2_2 generated based on the second ultrasonic reception signal UR2 received from the measurement site MR may be illustrated as shown in FIG. 9, and the width of the measurement site MR shown in FIG. 9 may be A2.

The volume measurement unit 300 may provide the volume information VI of the measurement site MR based on the first ultrasonic image UI1 generated according to the first ultrasonic reception signal UR1 received by the first ultrasonic array 110 and the second ultrasonic image UI2 generated according to the second ultrasonic reception signal UR2 received by the second ultrasonic array 120. For example, the volume information VI of the measurement site MR may be identified using parameters including the height and width of the measurement site MR included in the 1_1 ultrasonic image U1_1 and the width and the steering angle of the measurement site included in the 2_1 ultrasonic image U2_1.

In an embodiment, the volume measurement unit 300 may calculate maximum area information in which the area of the measurement site MR is maximum based on the first ultrasonic image UI1 provided at each array position and calculate maximum width information in which the width of the measurement site MR is maximum based on the second ultrasonic image UI2 corresponding to the first ultrasonic image UI1 to provide the volume information VI of the measurement site M4. For example, when the center of the first ultrasonic array 110 is located at the second point P2, the height and width of the measurement site MR included in the 1_2 ultrasonic image U1_2 may be maximum and the width of the measurement site MR included in the 2_2 ultrasonic image U2_2 may be maximum. In this case, the volume measurement unit 300 may provide the maximum volume information VI of the measurement site MR using parameters including the height and width of the measurement site MR included in the 1_2 ultrasonic image U1_2 and the width and steering angle of the measurement site MR included in the 2_2 ultrasonic image U2_2. The parameters may be used to correct the width of the measurement site MR included in the 2_2 ultrasonic image U2_2.

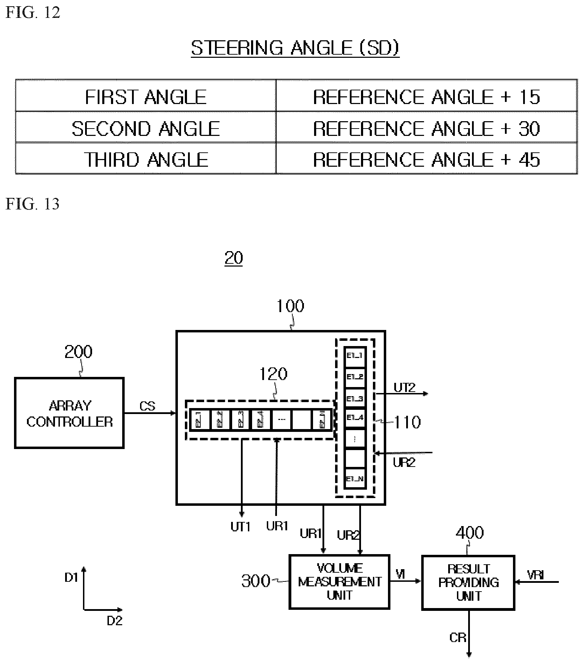

FIG. 10 is a diagram illustrating a steering angle applied to the ultrasonic volume measurement device of FIG. 1, FIG. 11 is a diagram illustrating an operating mode of the ultrasonic volume measurement device of FIG. 1, and FIG. 12 is a diagram illustrating an example of a steering angle applied to the ultrasonic volume measurement device of FIG. 1.

Referring to FIGS. 1 to 12, in an embodiment, an angle between a first straight line LN1 connecting the first ultrasonic image UI1 corresponding to the maximum area information from the array position to the maximum image point MP corresponding to the maximum image depth MD and a second straight line LN2 connecting the center CA1 of the second ultrasonic array 120 to the maximum image point MP may be the reference angle RD, and the steering angle SD may be greater than the reference angle RD. For example, in order to acquire an image including the measurement site MR using the second ultrasonic array 120, the delay applied to multiple second directional elements included in the second ultrasonic array 120 may change. When the delay applied to the second directional elements changes, the steering angle SD of the second ultrasonic array 120 may be changed. When the steering angle SD of the second ultrasonic array 120 is set to be greater than the reference angle RD, a larger second directional ultrasonic image around the measurement site MR may be acquired. In an embodiment, the array position may be set based on the center of the first ultrasonic array 110.

In an embodiment, an operating mode OM of the ultrasonic volume measurement device may include a scan mode SCM and a search mode SEM. In the scan mode SCM, maximum area information of the measurement site MR included in the image region may be calculated according to the first ultrasonic image UI1 provided using the first ultrasonic array 110. In the search mode SEM, if the same position as the maximum width information is detected while scanning using the first ultrasonic array 110, the maximum area information of the measurement site MR may be calculated using the second ultrasonic array 120.

For example, while the ultrasonic volume measurement device 10 operates in the scan mode SCM, the ultrasonic volume measurement device 10 may scan the height and width of the measurement site MR and calculate area information using the first ultrasonic array 110, while moving to the first point P1, the second point P2, and the third point P3. The area information of the first point P1 to the third point P3 may be stored in a database. Thereafter, the ultrasonic volume measurement device 10 may operate in the search mode SEM. While the ultrasonic volume measurement device 10 is operating in search mode SEM, when a second point P2 corresponding to the same position as the maximum area information is detected while scanning using the first ultrasonic array 110, the ultrasonic volume measurement device 10 may transmit the second ultrasonic transmission signal UT2 to the measurement site MR using the second ultrasonic array 120 and calculate maximum width information of the measurement site MR based on the second ultrasonic reception signal UR2 received from the measurement site MR.

In an embodiment, the steering angle SD may be set to sequentially increase or decrease according to a predetermined angle interval. For example, when the steering angle SD is set to a first angle, the steering angle SD may be set as an angle obtained by adding 15 degrees to the reference angle RD, and when the steering angle SD is set to a second angle, the steering angle SD may be set as an angle obtained by adding 30 degrees to the reference angle RD. In addition, when the steering angle SD is set to a third angle, the steering angle SD may be set as an angle obtained by adding 45 degrees to the reference angle RD. A user of the ultrasonic volume measurement device 10 according to the present disclosure may adjust the steering angle SD to acquire an appropriate image of the surrounding area including the measurement site MR.

The ultrasonic volume measurement device 10 according to the present disclosure may include multiple second directional elements arranged in the second direction D2 perpendicular to the first direction D1 in which the first ultrasonic array 110 is disposed, and may accurately identify the volume information VI of the measurement site MR included in the image region using the second ultrasonic array 120 disposed on one side SD1 of the first ultrasonic array 110.

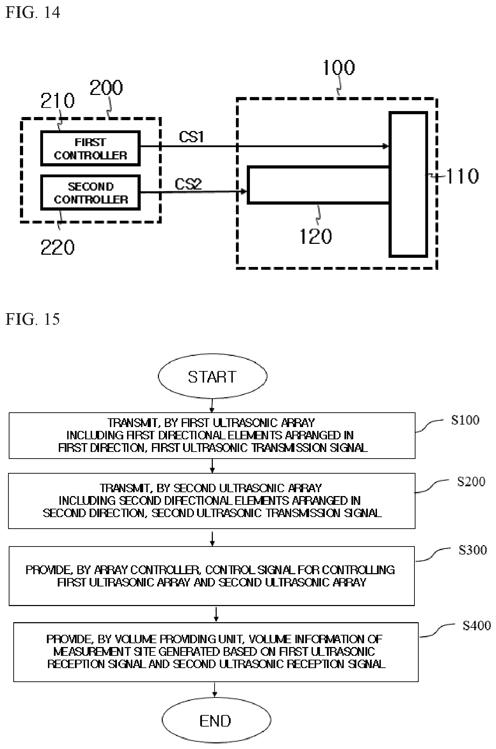

FIG. 13 is a diagram illustrating an ultrasonic imaging device according to embodiments of the present disclosure, and FIG. 14 is a diagram illustrating an operation of an array controller included in the ultrasonic imaging device of FIG. 13.

Referring to FIGS. 1 to 14, the ultrasonic volume measurement system according to an embodiment of the present disclosure may include a first ultrasonic array 110, a second ultrasonic array 120, an array controller 200, a volume measurement unit 300, and a result providing unit 400. The first ultrasonic array 110 may include multiple first directional elements arranged in the first direction D1 and may transmit the first ultrasonic transmission signal UT1. The second ultrasonic array 120 may include multiple second directional elements arranged in the second direction D2 perpendicular to the first direction D1, transmit the second ultrasonic transmission signal UT2, and may be disposed on one side SD1 of the first ultrasonic array 110. The array controller 200 may provide control signals for controlling the first ultrasonic array 110 and the second ultrasonic array 120. The volume measurement unit 300 may provide the volume information VI of the measurement site MR included in the image region generated based on the first ultrasonic reception signal UR1 received by the first ultrasonic array 110 and the second ultrasonic reception signal UR2 received by the second ultrasonic array 120. The result providing unit 400 may compare the volume information VI of the measurement site MR with a predetermined volume reference information VRI and provide a comparison result CR.

In an embodiment, the array controller 200 may include a first controller 210 and a second controller 220. The first controller 210 may provide a first control signal CS1 for controlling the first directional elements included in the first ultrasonic array 110. The second controller 220 may provide a second control signal CS2 for controlling the second directional elements included in the second ultrasonic array 120. For example, the first controller 210 may provide the first ultrasonic transmission signal UT1 by driving only the odd-numbered elements, among the 1_1 element E1_1 and the 1_2 element E1_2 to the 1_N elements E1_N corresponding to multiple first directional elements based on the first control signal CS1, and may provide the first ultrasonic transmission signal UT1 by turning off some elements on both sides based on the center of the first ultrasonic array, among the 1_1 element E1_1 and the 1_2 element E1_2 to the 1_N element E1_N corresponding to multiple first directional elements.

The second controller 220 may provide the second control signal CS2 for controlling the second directional elements included in the second ultrasonic array 120. For example, the second controller 220 may provide the second ultrasonic transmission signal UT2 by driving only the even-numbered elements, among the 2_1 element E2_1 and the 2_2 element to the 2_N element E2_N corresponding to multiple second directional elements based on the second control signal CS2, and may provide the second ultrasonic transmission signal UT2 by turning off some elements on both sides based on the center of the second ultrasonic array, among the 2_1 element E2_1 and the 2_2 element to the 2_N element E2_N corresponding to multiple second directional elements.

FIG. 15 is a flowchart illustrating an operating method an ultrasonic device according to embodiments of the present disclosure.

Referring to FIGS. 1 to 15, in the operating method of the ultrasonic volume measurement device 10 according to an embodiment of the present disclosure, the first ultrasonic array 110 including multiple first directional elements arranged in the first direction D1 may transmit the first ultrasonic transmission signal UT1 (S100). The second ultrasonic array 120 including multiple second directional elements arranged in the second direction D2 perpendicular to the first direction D1 may transmit the second ultrasonic transmission signal UT2 (S200). The array controller 200 may provide a control signal to control the first ultrasonic array 110 and the second ultrasonic array 120 (S300). The volume measurement unit 300 may provide the volume information VI of the measurement site MR included in the image region generated based on the first ultrasonic reception signal UR1 received by the first ultrasonic array 110 and the second ultrasonic reception signal UR2 received by the second ultrasonic array 120 (S400).

In an embodiment, when the first ultrasonic array 110 and the second ultrasonic array 120 are disposed at a scanning position of the human body, the first ultrasonic reception signal UR1 and the second ultrasonic reception signal UR2 may be received by sequentially alternately turning on the first ultrasonic array 110 and the second ultrasonic array 120.

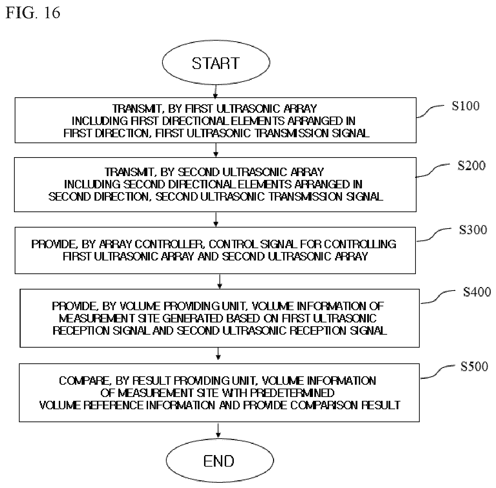

FIG. 16 is a flowchart illustrating an operating method of an ultrasonic imaging device according to embodiments of the present disclosure.

Referring to FIGS. 1 to 16, in the operating method of the ultrasonic volume measurement system according to an embodiment of the present disclosure, the first ultrasonic array 110 including multiple first directional elements arranged in the first direction D1 may transmit the first ultrasonic transmission signal UT1 (S100). The second ultrasonic array 120 including multiple second directional elements arranged in the second direction D2 perpendicular to the first direction D1 may transmit the second ultrasonic transmission signal UT2 (S200). The array controller 200 may provide a control signal to control the first ultrasonic array 110 and the second ultrasonic array 120 (S300). The volume measurement unit 300 may provide the volume information VI of the measurement site MR included in the image region generated based on the first ultrasonic reception signal UR1 received by the first ultrasonic array 110 and the second ultrasonic reception signal UR2 received by the second ultrasonic array 120 (S400). The result providing unit 400 may compare the volume information VI of the measurement site MR with the predetermined volume reference information VRI and provide the comparison result CR (S500).

In the operating method of the ultrasonic volume measurement system according to the present disclosure, multiple second directional elements arranged in the second direction D2 perpendicular to the first direction D1 in which the first ultrasonic array 110 is disposed may be included, and the volume information VI of the measurement site MR included in the image region may be accurately identified using the second ultrasonic array 120 disposed on one side SD1 of the first ultrasonic array 110.

## Claims

1. An ultrasonic volume measurement device (10) comprising:
a first ultrasonic array (110) including multiple first transducer elements (E1_1, ..., E1_N) arranged in a first direction (D1) and transmitting a first ultrasonic transmission signal (UT1);
a second ultrasonic array (120) including multiple second transducer elements (E2_1, ..., E2_N) disposed in a second direction (D2) perpendicular to the first direction (D1), transmitting a second ultrasonic transmission signal (UT2);
an array controller (200) providing control signals (CS1, CS2) to control the first ultrasonic array (110) and the second ultrasonic array (120); and
a volume measurement unit (300) providing volume information (VI) of a measurement site (MR) included in an image region, wherein the image region is generated based on a first ultrasonic reception signal (UR1) received by the first ultrasonic array (110) and a second ultrasonic reception signal (UR2) received by the second ultrasonic array (120),
**characterised in that**,
when the first ultrasonic array (110) and the second ultrasonic array (120) are arranged at a scanning position of a human body, the first ultrasonic array (110) and the second ultrasonic array (120) are sequentially turned on to receive the first ultrasonic reception signal (UR1) and the second ultrasonic reception signal (UR2), such that:
during a first time interval (TI1), the first ultrasonic array (110) is driven based on a first control signal (CS1) to transmit the first ultrasonic transmission signal (UT1) and receive the first ultrasonic reception signal (UR1) reflected from the measurement site (MR), while the second ultrasonic array (120) is turned off based on a second control signal (CS2), and
during a second time interval (TI2), the first ultrasonic array (110) is turned off based on the first control signal (CS1), while the second ultrasonic array (120) is driven based on the second control signal (CS2) to transmit the second ultrasonic transmission signal (UT2) and receive the second ultrasonic reception signal (UR2) reflected from the measurement site (MR).

2. The ultrasonic volume measurement device (10) of claim 1, wherein the second ultrasonic array (120) transmits the second ultrasonic transmission signal (UT2) having a steering angle (SD) to the measurement site (MR) included in the image region and receives the second ultrasonic reception signal (UR2) reflected from the measurement site (MR).

3. The ultrasonic volume measurement device (10) of claim 2, further comprising an image providing unit (310) providing a first ultrasonic image (UI1) corresponding to the first ultrasonic reception signal (UR1) and a second ultrasonic image (UI2) corresponding to the second ultrasonic reception signal (UR2) at each of array positions (P1, P2, P3) in which the first ultrasonic array (110) and the second ultrasonic array (120) are arranged.

4. The ultrasonic volume measurement device (10) of claim 2, wherein the volume measurement unit (300) calculates maximum area information in which an area of the measurement site (MR) is maximum based on the first ultrasonic images (UI1) provided at each of array positions (P1, P2, P3) and calculates maximum width information in which a width of the measurement site (MR) is maximum based on the second ultrasonic images (UI2) corresponding to the first ultrasonic images (UI1) to provide volume information (VI) of the measurement site (MR). [54-57, fig. 3]

5. The ultrasonic volume measurement device (10) of claim 4, wherein an angle between a first straight line (LN1) connecting the array position (P2) to a maximum image point (MP) corresponding to a maximum image depth (MD) in the first ultrasonic image (UI1) corresponding to the maximum area information and a second straight line (LN2) connecting a center (CA1) of the second ultrasonic array (120) to the maximum image point (MP) is a reference angle (RD), and the steering angle (SD) is greater than the reference angle (RD).

6. The ultrasonic volume measurement device (10) of claim 4, wherein an operating mode of the ultrasonic volume measurement device (10) includes a scan mode and a search mode,
in the scan mode, the maximum area information of the measurement site (MR) included in the image region is calculated according to a first ultrasonic image (UI1) provided using the first ultrasonic array (110) while moving to the array positions (P1, P2, P3), wherein area information at each of the array positions (P1, P2, P3) is stored in a database, and
in the search mode performed after the scan mode, the maximum width information of the measurement site (MR) is calculated using the second ultrasonic array (120), when the same position as the maximum area information is detected, while scanning using the first ultrasonic array (110).

7. The ultrasonic volume measurement device (10) of claim 6, wherein the steering angle (SD) is settable to increase or decrease sequentially according to a predetermined angle interval.

8. An ultrasonic volume measurement system (20) comprising:
the ultrasonic volume measurement device (10) according to any one of claims 1 to 7; and
a result providing unit (400) comparing the volume information (VI) of the measurement site (MR) with predetermined volume reference information and providing a comparison result.

9. The ultrasonic volume measurement system of claim 10, wherein
the array controller (200) includes:
a first controller providing the first control signal (CS1) to control the first transducer elements (E1_1, ..., E1_N) included in the first ultrasonic array (110); and
a second controller providing the second control signal (CS2) to control the second transducer elements (E2_1, ..., E2_N) included in the second ultrasonic array (120).

10. An operating method of an ultrasonic volume measurement device (10), the operating method comprising:
transmitting a first ultrasonic transmission signal (UT1) by a first ultrasonic array (110) including multiple first transducer elements (E1_1, ..., E1_N) arranged in a first direction (D1);
transmitting a second ultrasonic transmission signal (UT2) by a second ultrasonic array (120) including multiple second transducer elements (E2_1, ..., E2_N) arranged in a second direction (D2) perpendicular to the first direction (D1);
providing, by an array controller (200), a control signal to control the first ultrasonic array (110) and the second ultrasonic array (120); and
providing, by a volume measurement unit (300), volume information (VI) of the measurement site (MR) included in an image region, wherein the image region is generated based on a first ultrasonic reception signal (UR1) received by the first ultrasonic array (110) and a second ultrasonic reception signal (UR2) received by the second ultrasonic array (120),
**characterised in that**,
when the first ultrasonic array (110) and the second ultrasonic array (120) are arranged at a scanning position of a human body, the first ultrasonic array (110) and the second ultrasonic array (120) are sequentially turned on to receive the first ultrasonic reception signal (UR1) and the second ultrasonic reception signal (UR2) by:
during a first time interval (TI1), driving the first ultrasonic array (110) based on a first control signal (CS1) to transmit the first ultrasonic transmission signal (UT1) and receive the first ultrasonic reception signal (UR1) reflected from the measurement site (MR), while turning off the second ultrasonic array (120) based on a second control signal (CS2), and
during a second time interval (TI2), turning off the first ultrasonic array (110) based on the first control signal (CS1), while deriving the second ultrasonic array (120) based on the second control signal (CS2) to transmit the second ultrasonic transmission signal (UT2) and receive the second ultrasonic reception signal (UR2) reflected from the measurement site (MR).

11. An operating method of an ultrasonic volume measurement system (20), the operating method comprising:
the operating method according to claim 10; and
comparing, by a result providing unit (400), the volume information (VI) of the measurement site (MR) and predetermined volume reference information and providing a comparison result.

12. The operating method of claim 11, wherein the second ultrasonic array (120) transmits the second ultrasonic transmission signal (UT2) having a steering angle (SD) to the measurement site (MR) included in the image region and receives the second ultrasonic reception signal (UR2) reflected from the measurement site (MR).

## Patentansprüche

1. Ultraschallvolumenmessvorrichtung (10), umfassend:
ein erstes Ultraschallarray (110), das mehrere erste Wandlerelemente (E1_1, ..., E1_N) umfasst, die in einer ersten Richtung (D1) angeordnet sind, und ein erstes Ultraschallsendesignal (UT1) sendet;
ein zweites Ultraschallarray (120), das mehrere zweite Wandlerelemente (E2_1, ..., E2_N) umfasst, die in einer zweiten Richtung (D2) senkrecht zu der ersten Richtung (D1) angeordnet sind, und ein zweites Ultraschallsendesignal (UT2) sendet;
eine Arraysteuereinheit (200), die Steuersignale (CS1, CS2) zum Steuern des ersten Ultraschallarrays (110) und des zweiten Ultraschallarrays (120) bereitstellt; und
eine Volumenmesseinheit (300), die Volumeninformationen (VI) einer Messstelle (MR), die in einem Bildbereich enthalten ist, bereitstellt, wobei der Bildbereich auf Grundlage eines ersten Ultraschallempfangssignals (UR1), das von dem ersten Ultraschallarray (110) empfangen wird, und eines zweiten Ultraschallempfangssignals (UR2), das von dem zweiten Ultraschallarray (120) empfangen wird, erzeugt wird,
**dadurch gekennzeichnet, dass**,
wenn das erste Ultraschallarray (110) und das zweite Ultraschallarray (120) an einer Abtastposition eines menschlichen Körpers angeordnet sind, das erste Ultraschallarray (110) und das zweite Ultraschallarray (120) nacheinander eingeschaltet werden, um das erste Ultraschallempfangssignal (UR1) und das zweite Ultraschallempfangssignal (UR2) zu empfangen, sodass:
während eines ersten Zeitintervalls (TI1) das erste Ultraschallarray (110) auf Grundlage eines ersten Steuersignals (CS1) angesteuert wird, um das erste Ultraschallsendesignal (UT1) zu senden und das von der Messstelle (MR) reflektierte erste Ultraschallempfangssignal (UR1) zu empfangen, während das zweite Ultraschallarray (120) auf Grundlage eines zweiten Steuersignals (CS2) ausgeschaltet ist, und
während eines zweiten Zeitintervalls (TI2) das erste Ultraschallarray (110) auf Grundlage des ersten Steuersignals (CS1) ausgeschaltet ist, während das zweite Ultraschallarray (120) auf Grundlage des zweiten Steuersignals (CS2) angesteuert wird, um das zweite Ultraschallsendesignal (UT2) zu senden und das von der Messstelle (MR) reflektierte zweite Ultraschallempfangssignal (UR2) zu empfangen.

2. Ultraschallvolumenmessvorrichtung (10) nach Anspruch 1, wobei das zweite Ultraschallarray (120) das zweite Ultraschallsendesignal (UT2), das einen Schwenkwinkel (SD) aufweist, zu der in dem Bildbereich enthaltenen Messstelle (MR) sendet und das von der Messstelle (MR) reflektierte zweite Ultraschallempfangssignal (UR2) empfängt.

3. Ultraschallvolumenmessvorrichtung (10) nach Anspruch 2, ferner umfassend eine Bildbereitstellungseinheit (310), die ein erstes Ultraschallbild (UI1), das dem ersten Ultraschallempfangssignal (UR1) entspricht, und ein zweites Ultraschallbild (UI2), das dem zweiten Ultraschallempfangssignal (UR2) entspricht, an jeder der Arraypositionen (P1, P2, P3), an denen das erste Ultraschallarray (110) und das zweite Ultraschallarray (120) angeordnet sind, bereitstellt.

4. Ultraschallvolumenmessvorrichtung (10) nach Anspruch 2, wobei die Volumenmesseinheit (300) auf Grundlage der an jeder der Arraypositionen (P1, P2, P3) bereitgestellten ersten Ultraschallbilder (UI1) Informationen zur maximalen Fläche berechnet, bei der eine Fläche der Messstelle (MR) maximal ist, und auf Grundlage der zweiten Ultraschallbilder (UI2), die den ersten Ultraschallbildern (UI1) entsprechen, Informationen zur maximalen Breite berechnet, bei der eine Breite der Messstelle (MR) maximal ist, um Volumeninformationen (VI) der Messstelle (MR) bereitzustellen.

5. Ultraschallvolumenmessvorrichtung (10) nach Anspruch 4, wobei ein Winkel zwischen einer ersten geraden Linie (LN1), die die Arrayposition (P2) mit einem maximalen Bildpunkt (MP), der einer maximalen Bildtiefe (MD) in dem ersten Ultraschallbild (UI1) entspricht, das den Informationen zur maximalen Fläche entspricht, verbindet, und einer zweiten geraden Linie (LN2), die eine Mitte (CA1) des zweiten Ultraschallarrays (120) mit dem maximalen Bildpunkt (MP) verbindet, ein Referenzwinkel (RD) ist, und der Schwenkwinkel (SD) größer als der Referenzwinkel (RD) ist.

6. Ultraschallvolumenmessvorrichtung (10) nach Anspruch 4, wobei ein Betriebsmodus der Ultraschallvolumenmessvorrichtung (10) einen Abtastmodus und einen Suchmodus umfasst,
wobei in dem Abtastmodus die Informationen zur maximalen Fläche der in dem Bildbereich enthaltenen Messstelle (MR) gemäß einem ersten Ultraschallbild (UI1) berechnet werden, das unter Verwendung des ersten Ultraschallarrays (110) bereitgestellt wird, während dieses zu den Arraypositionen (P1, P2, P3) bewegt wird, wobei Flächeninformationen an jeder der Arraypositionen (P1, P2, P3) in einer Datenbank gespeichert werden, und
wobei in dem nach dem Abtastmodus durchgeführten Suchmodus die Informationen zur maximalen Breite der Messstelle (MR) unter Verwendung des zweiten Ultraschallarrays (120) berechnet werden, wenn dieselbe Position wie diejenige der Informationen zur maximalen Fläche detektiert wird, während unter Verwendung des ersten Ultraschallarrays (110) abgetastet wird.

7. Ultraschallvolumenmessvorrichtung (10) nach Anspruch 6, wobei der Schwenkwinkel (SD) derart einstellbar ist, dass er gemäß einem vorbestimmten Winkelintervall schrittweise vergrößert oder verkleinert wird.

8. Ultraschallvolumenmesssystem (20), umfassend:
die Ultraschallvolumenmessvorrichtung (10) nach einem der Ansprüche 1 bis 7; und
eine Ergebnisbereitstellungseinheit (400), die die Volumeninformationen (VI) der Messstelle (MR) mit vorbestimmten Volumenreferenzinformationen vergleicht und ein Vergleichsergebnis bereitstellt.

9. Ultraschallvolumenmesssystem nach Anspruch 8, wobei
die Arraysteuereinheit (200) umfasst:
eine erste Steuereinheit, die das erste Steuersignal (CS1) zum Steuern der in dem ersten Ultraschallarray (110) enthaltenen ersten Wandlerelemente (E1_1, ..., E1_N) bereitstellt; und
eine zweite Steuereinheit, die das zweite Steuersignal (CS2) zum Steuern der in dem zweiten Ultraschallarray (120) enthaltenen zweiten Wandlerelemente (E2_1, ..., E2_N) bereitstellt.

10. Betriebsverfahren einer Ultraschallvolumenmessvorrichtung (10), wobei das Betriebsverfahren umfasst:
Senden eines ersten Ultraschallsendesignals (UT1) durch ein erstes Ultraschallarray (110), das mehrere erste Wandlerelemente (E1_1, ..., E1_N) umfasst, die in einer ersten Richtung (D1) angeordnet sind;
Senden eines zweiten Ultraschallsendesignals (UT2) durch ein zweites Ultraschallarray (120), das mehrere zweite Wandlerelemente (E2_1, ..., E2_N) umfasst, die in einer zweiten Richtung (D2) senkrecht zu der ersten Richtung (D1) angeordnet sind;
Bereitstellen, durch eine Arraysteuereinheit (200), eines Steuersignals zum Steuern des ersten Ultraschallarrays (110) und des zweiten Ultraschallarrays (120); und
Bereitstellen, durch eine Volumenmesseinheit (300), von Volumeninformationen (VI) der in einem Bildbereich enthaltenen Messstelle (MR), wobei der Bildbereich auf Grundlage eines ersten Ultraschallempfangssignals (UR1), das von dem ersten Ultraschallarray (110) empfangen wird, und eines zweiten Ultraschallempfangssignals (UR2), das von dem zweiten Ultraschallarray (120) empfangen wird, erzeugt wird,
**dadurch gekennzeichnet, dass**,
wenn das erste Ultraschallarray (110) und das zweite Ultraschallarray (120) an einer Abtastposition eines menschlichen Körpers angeordnet sind, das erste Ultraschallarray (110) und das zweite Ultraschallarray (120) nacheinander eingeschaltet werden, um das erste Ultraschallempfangssignal (UR1) und das zweite Ultraschallempfangssignal (UR2) zu empfangen, durch:
während eines ersten Zeitintervalls (TI1), Ansteuern des ersten Ultraschallarrays (110) auf Grundlage eines ersten Steuersignals (CS1), um das erste Ultraschallsendesignal (UT1) zu senden und das von der Messstelle (MR) reflektierte erste Ultraschallempfangssignal (UR1) zu empfangen, während das zweite Ultraschallarray (120) auf Grundlage eines zweiten Steuersignals (CS2) ausgeschaltet wird, und
während eines zweiten Zeitintervalls (TI2), Ausschalten des ersten Ultraschallarrays (110) auf Grundlage des ersten Steuersignals (CS1), während das zweite Ultraschallarray (120) auf Grundlage des zweiten Steuersignals (CS2) angesteuert wird, um das zweite Ultraschallsendesignal (UT2) zu senden und das von der Messstelle (MR) reflektierte zweite Ultraschallempfangssignal (UR2) zu empfangen.

11. Betriebsverfahren eines Ultraschallvolumenmesssystems (20), wobei das Betriebsverfahren umfasst:
das Betriebsverfahren nach Anspruch 10; und
Vergleichen der Volumeninformationen (VI) der Messstelle (MR) mit vorbestimmten Volumenreferenzinformationen und Bereitstellen eines Vergleichsergebnisses durch eine Ergebnisbereitstellungseinheit (400).

12. Betriebsverfahren nach Anspruch 11, wobei das zweite Ultraschallarray (120) das zweite Ultraschallsendesignal (UT2), das einen Schwenkwinkel (SD) aufweist, zu der in dem Bildbereich enthaltenen Messstelle (MR) sendet und das von der Messstelle (MR) reflektierte zweite Ultraschallempfangssignal (UR2) empfängt.

## Revendications

1. Dispositif de mesure de volume (10) par ultrasons comprenant:
un premier réseau ultrasonore (110) comprenant plusieurs premiers éléments transducteurs (E1_1, ..., E1_N) disposés selon une première direction (D1) et émettant un premier signal ultrasonore d'émission (UT1);
un deuxième réseau ultrasonore (120) comprenant plusieurs deuxièmes éléments transducteurs (E2_1, ..., E2_N) disposés selon une deuxième direction (D2) perpendiculaire à la première direction (D1), émettant un deuxième signal ultrasonore d'émission (UT2);
un contrôleur de réseau (200) fournissant des signaux de commande (CS1, CS2) pour commander le premier réseau ultrasonore (110) et le deuxième réseau ultrasonore (120); et
une unité de mesure de volume (300) fournissant des informations de volume (VI) d'un site de mesure (MR) inclus dans une région d'image, la région d'image étant générée à partir d'un premier signal ultrasonore de réception (UR1) reçu par le premier réseau ultrasonore (110) et d'un deuxième signal ultrasonore de réception (UR2) reçu par le deuxième réseau ultrasonore (120),
**caractérisé en ce que**, lorsque le premier réseau ultrasonore (110) et le deuxième réseau ultrasonore (120) sont disposés à une position de balayage d'un corps humain, le premier réseau ultrasonore (110) et le deuxième réseau ultrasonore (120) sont activés successivement pour recevoir le premier signal ultrasonore de réception (UR1) et le deuxième signal ultrasonore de réception (UR2), de telle sorte que:
pendant un premier intervalle de temps (TI1), le premier réseau ultrasonore (110) est activé en fonction d'un premier signal de commande (CS1) afin d'émettre le premier signal ultrasonore d'émission (UT1) et de recevoir le premier signal ultrasonore de réception (UR1) réfléchi par le site de mesure (MR), tandis que le deuxième réseau ultrasonore (120) est désactivé en fonction d'un deuxième signal de commande (CS2), et
pendant un deuxième intervalle de temps (TI2), le premier réseau ultrasonore (110) est désactivé en fonction du premier signal de commande (CS1), tandis que le deuxième réseau ultrasonore (120) est activé en fonction du deuxième signal de commande (CS2) afin d'émettre le deuxième signal d'émission ultrasonore (UT2) et de recevoir le deuxième signal de réception ultrasonore (UR2) réfléchi par le site de mesure (MR).

2. Dispositif de mesure de volume par ultrasons (10) selon la revendication 1, dans lequel le deuxième réseau d'émetteurs ultrasonores (120) émet le deuxième signal ultrasonore d'émission (UT2), présentant un angle de direction (SD), vers le site de mesure (MR) inclus dans la région d'image, et reçoit le deuxième signal ultrasonore de réception (UR2) réfléchi par le site de mesure (MR).

3. Dispositif de mesure de volume par ultrasons (10) selon la revendication 2, comprenant en outre une unité de génération d'images (310) fournissant une première image ultrasonore (UI1) correspondant au premier signal ultrasonore de réception (UR1) et une deuxième image ultrasonore (UI2) correspondant au deuxième signal ultrasonore de réception (UR2) à chacune des positions de réseau (P1, P2, P3) auxquelles sont disposés le premier réseau ultrasonore (110) et le deuxième réseau ultrasonore (120).

4. Dispositif de mesure de volume par ultrasons (10) selon la revendication 2, dans lequel l'unité de mesure de volume (300) calcule les informations de surface maximale dans laquelle une zone du site de mesure (MR) est maximale, sur la base des premières images ultrasonores (UI1) fournies à chacune des positions de réseau (P1, P2, P3) et calcule les informations de largeur maximale, la largeur du site de mesure (MR) étant maximale, sur la base des deuxièmes images ultrasonores (UI2) correspondant aux premières images ultrasonores (UI1), afin de fournir des informations de volume (VI) du site de mesure (MR).

5. Dispositif de mesure de volume par ultrasons (10) selon la revendication 4, dans lequel l'angle entre une première droite (LN1) reliant la position de réseau (P2) à un point d'image maximal (MP) correspondant à une profondeur d'image maximale (MD) dans la première image ultrasonore (UI1) correspondant aux informations de surface maximale, et une deuxième droite (LN2) reliant un centre (CA1) du deuxième réseau ultrasonore (120) au point d'image maximal (MP) est un angle de référence (RD), et l'angle de direction (SD) est supérieur à l'angle de référence (RD).

6. Dispositif de mesure de volume par ultrasons (10) selon la revendication 4, dans lequel un mode de fonctionnement du dispositif de mesure de volume (10) par ultrasons comprend un mode balayage et un mode recherche,
en mode balayage, les informations de surface maximale de la zone de mesure (MR) incluse dans la région d'image sont calculées à partir d'une première image ultrasonore (UI1) obtenue à l'aide du premier réseau ultrasonore (110) lors du déplacement vers les positions de réseau (P1, P2, P3), les informations de surface à chacune des positions de réseau (P1, P2, P3) étant stockées dans une base de données, et
en mode recherche, qui suit le mode balayage, la largeur maximale du site de mesure (MR) est calculée à l'aide du deuxième réseau ultrasonore (120), lorsque la même position que celle correspondant à la surface maximale est détectée, pendant le balayage à l'aide du premier réseau ultrasonore (110).

7. Dispositif de mesure de volume (10) par ultrasons selon la revendication 6, dans lequel l'angle de direction (SD) peut être réglé de manière à augmenter ou à diminuer successivement selon un intervalle angulaire prédéterminé.

8. Système de mesure de volume (20) par ultrasons comprenant:
le dispositif de mesure de volume (10) par ultrasons selon l'une quelconque des revendications 1 à 7; et
une unité de calcul de résultat (400) qui compare les informations de volume (VI) du site de mesure (MR) à des informations de référence de volume prédéterminées et obtient un résultat de comparaison.

9. Système de mesure de volume par ultrasons selon la revendication 8, dans lequel
le contrôleur de réseau (200) comprend:
un premier contrôleur fournissant le premier signal de commande (CS1) pour commander les premiers éléments transducteurs (E1_1, ..., E1_N) inclus dans le premier réseau ultrasonore (110); et
un deuxième contrôleur fournissant le deuxième signal de commande (CS2) pour commander les deuxièmes éléments transducteurs (E2_1, ..., E2_N) inclus dans le deuxième réseau ultrasonore (120).

10. Procédé de fonctionnement d'un dispositif de mesure de volume (10) par ultrasons, le procédé de fonctionnement comprenant:
l'émission d'un premier signal ultrasonore (UT1) par un premier réseau ultrasonore (110) comprenant plusieurs premiers éléments transducteurs (E1_1, ..., E1_N) disposés dans une première direction (D1);
l'émission d'un deuxième signal ultrasonore (UT2) par un deuxième réseau ultrasonore (120) comprenant plusieurs deuxièmes éléments transducteurs (E2_1, ..., E2_N) disposés dans une deuxième direction (D1) perpendiculaire à la première direction (D1);
la fourniture d'un contrôleur de réseau (200), d'un signal de commande pour commander le premier réseau ultrasonore (110) et le deuxième réseau ultrasonore (120); et
la fourniture, par une unité de mesure de volume (300), des informations de volume (VI) du site de mesure (MR) inclus dans une région d'image, la région d'image étant générée à partir d'un premier signal ultrasonore de réception (UR1) reçu par le premier réseau ultrasonore (110) et d'un deuxième signal ultrasonore de réception (UR2) reçu par le deuxième réseau ultrasonore (120),
**caractérisé en ce que**, lorsque le premier réseau ultrasonore (110) et le deuxième réseau ultrasonore (120) sont disposés à une position de balayage d'un corps humain, le premier réseau ultrasonore (110) et le deuxième réseau ultrasonore (120) sont activés successivement pour recevoir le premier signal ultrasonore de réception (UR1) et le deuxième signal ultrasonore de réception (UR2):
pendant un premier intervalle de temps (TI1), par activation du premier réseau ultrasonore (110) en fonction d'un premier signal de commande (CS1) afin d'émettre le premier signal ultrasonore d'émission (UT1) et de recevoir le premier signal ultrasonore de réception (UR1) réfléchi par le site de mesure (MR), tout en désactivant le deuxième réseau ultrasonore (120) en fonction d'un deuxième signal de commande (CS2), et
pendant un deuxième intervalle de temps (TI2), par désactivation du premier réseau ultrasonore (110) en fonction du premier signal de commande (CS1), tout en dérivant le deuxième réseau ultrasonore (120) en fonction du deuxième signal de commande (CS2) afin d'émettre le deuxième signal d'émission ultrasonore (UT2) et de recevoir le deuxième signal de réception ultrasonore (UR2) réfléchi par le site de mesure (MR).

11. Procédé de fonctionnement d'un système de mesure de volume (20) par ultrasons, le procédé de fonctionnement comprenant:
le procédé de fonctionnement selon la revendication 10 ; et
la comparaison, par une unité de calcul de résultat (400), des informations de volume (VI) du site de mesure (MR) et des informations de référence de volume prédéterminées, et l'obtention d'un résultat de comparaison.

12. Procédé de fonctionnement selon la revendication 11, dans lequel le deuxième réseau ultrasonore (120) émet le deuxième signal ultrasonore d'émission (UT2), présentant un angle de direction (SD), vers le site de mesure (MR) inclus dans la région d'image, et reçoit le deuxième signal ultrasonore de réception (UR2) réfléchi par le site de mesure (MR).
